(19) Europäisches Patentamt
European Patent Office
Office européen des brevets

(11) EP 1 795 195 A1

(12) **EUROPEAN PATENT APPLICATION**
published in accordance with Art. 158(3) EPC

(43) Date of publication:
**13.06.2007 Bulletin 2007/24**

(21) Application number: **05792021.7**

(22) Date of filing: **23.09.2005**

(51) Int Cl.:
***A61K 31/4439*** *(2006.01)* ***A61P 1/04*** *(2006.01)*

(86) International application number:
**PCT/CN2005/001545**

(87) International publication number:
**WO 2006/034632 (06.04.2006 Gazette 2006/14)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.09.2004 CN 200410081026**
**29.03.2005 CN 200510059391**
**20.06.2005 CN 200510077315**

(71) Applicant: **Jiangsu Hansen Pharmaceutical Co., Ltd.**
**Lianyungang, Jiangsu 222006 (CN)**

(72) Inventors:
- **ZHONG, Huijuan**
  **Lianyungang, Jiangsu 222002 (CN)**
- **LV, Aifeng**
  **Lianyungang, Jiangsu 222002 (CN)**

(74) Representative: **Ziebig, Marlene et al**
**Gulde Hengelhaupt Ziebig & Schneider**
**Wallstrasse 58/59**
**10179 Berlin (DE)**

(54) **NEW PRAZOLE COMPOUND AND THE USE THEREOF**

(57) The present invention relates to derivatives of compounds (formula (I) or (II)) and their salts, wherein $R_1$ represents lower alkyl or the lower alkyl substituted by halogen atoms, $R_2$ represents straight-chain or branched-chain alkyl containing 1-4 carbon atoms and $R_3$ represents hydrogen atom or alkali metals such as lithium, sodium, potassium or alkaline-earth metals such as magnesium and calcium. In the present invention, compounds with formula (I) or (II) and their derivatives or salts thereof acceptable in pharmacy and pharmacology can remarkably improve the effectiveness of anti-ulcer in alimentary tract, weakly inhibit gastric acid secretion, reduce the risk of stomach cancer and have excellent bio-availability and other in-vive pharmacokinetic characteristics.

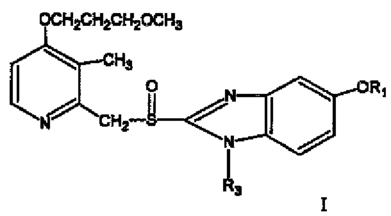

I

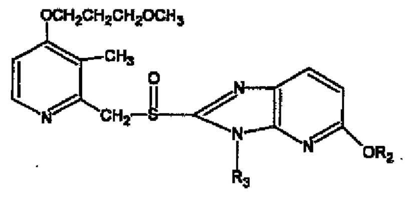

II

EP 1 795 195 A1

**Description**

FIELD OF THE INVENTION

The present invention relates to two kinds of derivatives of prazole compounds. The resulting compound products can be converted into pharmaceutically acceptable salts thereof by conventional process which are then used as active ingredients in medicament production.

BACKGROUND OF THE INVENTION

European patent application EP150 586 disclosed 2-(pyridyl methylthio- or sulfinyl-) benzimidazole, wherein $4^{th}$ position of the pyridine ring may be substituted by alkylthio or arylthio. This application revealed that the compound had a long-term and continuous inhibition against gastric acid secretion. An international application WO92/1296 recorded the compounds obtained by some specific substitutions of 2-(pyridyl methylthio- or sulfinyl-) benzimidazole which could effectively kill *Helicobacter* and these compounds could be used for preventing and curing gastric diseases. Another international application WO93/24480 also recorded other 2-(pyridyl methylthio- or sulfinyl-) benzimidazoles obtained by some specific substitutions which could effectively kill *Helicobacter.*

SUMMARY OF THE INVENTIN

The purpose of the present invention is to provide two kinds of Prazole derivatives or salts thereof.

To achieve the purpose of the invention, a derivative of the compound of formula (I) or (II) or a salt thereof is provided

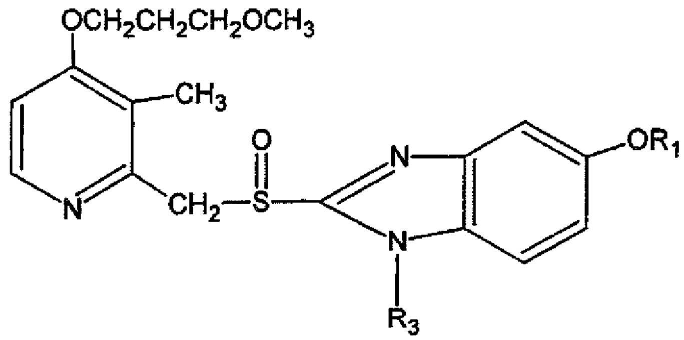

I

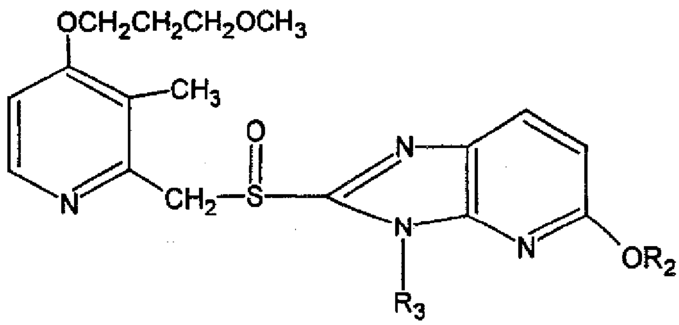

II

Wherein $R_1$ represents lower alkyl or halogen substituted lower alkyl, $R_2$ represents straight-chain or branched-chain alkyl having 1 to 4 carbon atoms, and $R_3$ represents hydrogen, alkali metal such as lithium, sodium or potassium, or alkaline-earth metal such as magnesium or calcium; a salt represented by formula (I) or (II) is a hemi-magnesium salt or a hemi-calcium salt when $R_3$ represents a alkali earth metal such as magnesium or calcium.

Preferably, $R_1$ is difluoromethyl and $R_3$ is sodium in formula (I); and $R_2$ is methyl and $R_3$ is sodium in formula (II).

The compound or the salt thereof according to the present invention is also a compound or a salt thereof in the form of single (R) or (S) enantiomer, or rich in an enantiomer.

The present invention also provides a pharmaceutical composition comprising said compound and/or the pharmaceutically acceptable salt.

In addition, the present invention discloses the use of said compound and/or the pharmaceutically acceptable salt thereof in preparation of a medicament for killing *Helicobacter pylori,* and the use in preparation of a medicament for treating and/or preventing gastric and/or intestinal diseases.

The compounds of formula (I) or (II) of the present invention and their derivatives or pharmaceutically or pharmacologically acceptable salts can improve remarkably the effect against alimentary tract ulcer, and weakly inhibit gastric acid secretion, which reduces greatly the risk of stomach cancer, and additionally have more excellent in vivo pharmacokinetic characteristics such as the bio-availability, and the like.

DESCRIPTION OF THE INVENTION

FIG. 1 shows a curve of plasma concentration against time after 5mg/kg tenatoprazole (T) and compound (II)(Y) were administered to six dogs.

FIG. 2 shows a curve of mean plasma concentration against time after 5mg/kg tenatoprazole (T) and compound (II)(Y) were administered to six dogs.

DETAILED DESCRIPTION OF THE INVENTION

The present invention will be illustrated detailedly by the following examples. The examples are provided for the purpose of illustration and are not meant to limit the scope of the present invention in any manner.

Compound (I) is taken as an example to indicate the synthetic route shown as follows;

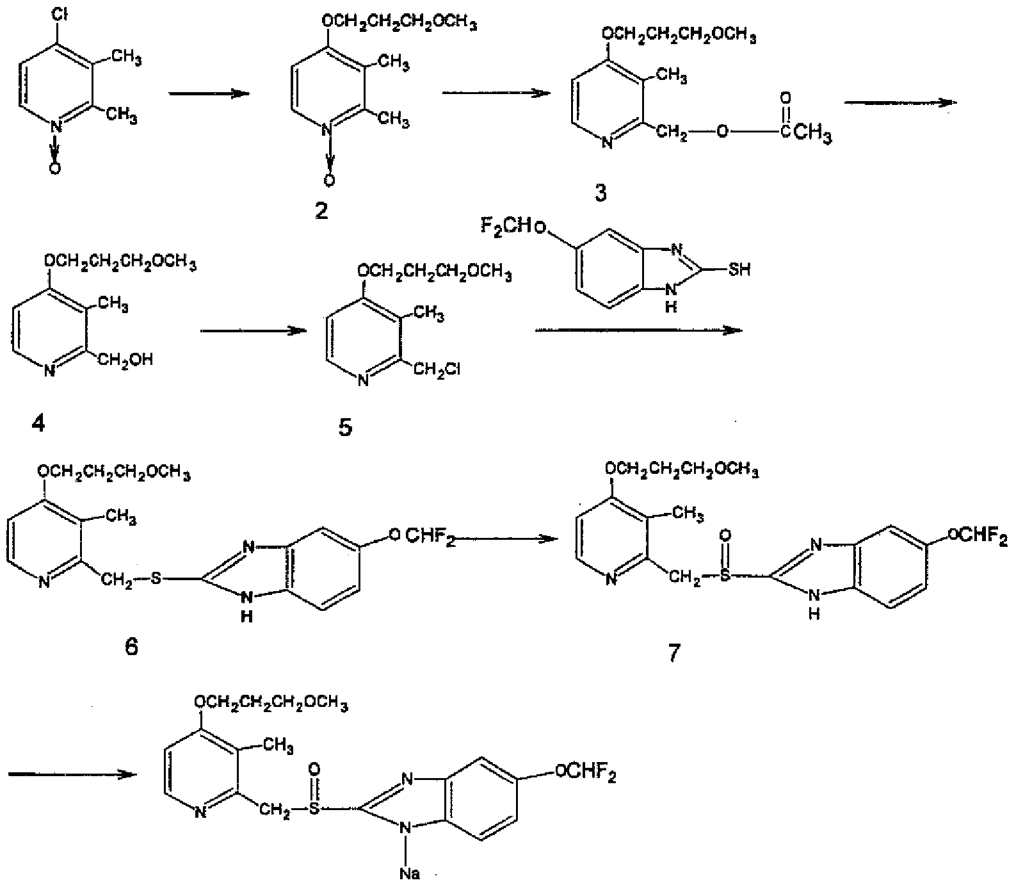

2

3

4

5

6

7

Intermediate (2) is prepared by the substitution reaction of 2, 3-dimethyl-4-Cl-pyridine N-oxide with sodium 3-methoxyl-1-propanoxide, and the latter is obtained by the reaction of 3-methoxyl-1-propanol with strong alkali. Said strong alkali includes sodium, sodium-hydride (60%-80%), sodamide, etc., which takes place in a solvent such as anhydrous tetrahydrofuran, N,N-dimethyl formamide, and dimethyl sulfoxide, and anhydrous tetrahydrofuran is preferred. The temperature for the reaction is between 50 °C and 150 °C, and about 100 °C is preferred.

Intermediate (3) is prepared by the reaction of 2, 3-dimethyl-4-(3-methoxyl-propoxyl) -pyridine N-oxide with acid anhydride or acyl chloride, such as acetyl chloride, trichloroacetyl chloride, and trifluoroacetyl chloride, and acid anhydride is preferred. The reaction may take place in a solvent such as acetonitrile, benzene, chloroform, dichloromethane, dimethyl formamide, dimethyl sulfoxide, dioxane, tetrahydrofuran, and toluene, etc., and acetonitrile is preferred. The temperature for the reaction is from the ambient temperature to 150 °C, and 80-100 °C is preferred. The reaction time is from 2 to 10 hours. After the reaction, the reaction solution is concentrated under vacuum to evaporate the solvent, and the resulting product is used in the next step.

Intermediate (4) is obtained from intermediate (3) by hydrolysis. The reaction may take place in a polar solvent containing hydroxyl such as water, methanol, ethanol, isopropanol or butanol, and water or methanol is preferred. The alkali used in this step is sodium hydroxide, potassium hydroxide, potassium carbonate, ammonia or sodium methoxide, etc., and sodium hydroxide is preferred. The reaction temperature may be controlled between 20-80 °C, and preferably between 30-50 °C. The reaction time may be controlled between 1-10 hours. After the reaction, the reaction solution is extracted by an organic solvent, such as dichloromethane, chloroform, ethyl ether, tert-butyl methyl ether, ethyl acetate, toluene, and isopropyl ether, and chloroform is preferred. After the organic layer is dried, the solvent is recovered under vacuum till the residue is dry, and the resulting product is then directly used in the next step.

Intermediate (5) is prepared by the reaction of the intermediate obtained from the preceding step with a halide reagent, such as sulfoxide halide or phosphorus trihalide, and sulfoxide chloride is preferred. The reaction takes place in an inert solvent, such as acetonitrile, benzene, chloroform, dichloromethane, dimethyl formamide, dimethyl sulfoxide and tetrahydrofuran, etc., and dichloromethane or chloroform is preferred. The reaction temperature may be controlled between -10 °C and the temperature of solvent reflux, and the temperature between 0-25 °C is preferred. After the reaction, the reaction solution is neutralized by alkali, such as sodium hydroxide, potassium hydroxide, sodium carbonate, and potassium carbonate, etc., and sodium carbonate is preferred. The pH value is adjusted to 7-8. The solution is

extracted by an organic solvent such as dichloromethane, chloroform, ethyl ether and ethyl acetate, and chloroform or dichloromethane is preferred. The organic phase is dried and then concentrated under vacuum. The resulting product is directly used in the next step.

Intermediate (6) is obtained by the condensation reaction of 2-chloromethyl-3-methyl-4-(3-methoxyl-propoxyl)-pyridine with 5-difluoromethoxyl-2-mercapto benzimidazole. The reaction can take place under the condition of liquid-liquid phase transfer. The inorganic alkali used includes sodium hydroxide or potassium hydroxide, etc.. The phase transfer catalyst may be $R_4NX$, $R_4PX$ or crown ether, etc., and $R_4NX$ is preferred. $R_4NX$ includes tetrabutyl ammonium, tetraethyl ammonium, and triethyl phenyl ammonium, etc., and X represents chlorine, bromine or iodine, etc., and triethyl phenyl ammonium chloride is preferred. The temperature for the reaction is 0-100 °C, and 15-30 °C is preferred. The reaction time is between 1-10 hours. The reaction may also take place in a homogeneous phase with polar solvent such as water, methanol, ethanol, iso-propanol and n-butanol, etc.. Under these conditions, 5-difluoromethoxy-2-mercapto-benzimidazole salt is firstly formed, and then reacts with intermediate (5) to produce intermediate (6).

Intermediate (7) is prepared by the reaction of the intermediate obtained from the preceding step with an oxidant, such as m-chloro-peroxybenzoic acid, peracetic acid, and hydrogen peroxide, etc., and m-cloro-peroxybenzoic acid is preferred. The reaction temperature is from -80 °C to 0 °C, and -50 °C-40 °C is preferred.

The derivative rich in (R) or (S) enantiomer can be obtained by asymmetry oxidation of Intermediate (6). The asymmetry oxidation is carried out by known processes, such as the process mentioned in Patent WO96/02535. Alternatively, the derivative can be separated from the compound of formula (I) or (II) by chiral resolution or chiral column . The present invention will be described in detail by following examples:

### Example 1

#### Preparation of 4- (3-methoxy-propoxy)-2,3-dimethyl-pyridine N-oxide

7.8g sodium was suspended in 90ml 3-methoxy-1- propanol, and then the suspension was heated up to 100 °C by oil bath until it turned clear completely. After cooled, 120ml THF and 30g 2,3-dimethyl-4-chloro-pyridine N-oxide were added and then the reflux reaction took place for 12 hours and was cooled again. 200ml water was added and pH value was adjusted to 7. The reaction solution was extracted with chloroform, dried with anhydrous magnesium sulfate and filtered. The filtrate was concentrated under vacuum to recover chloroform and 3-methoxy-1-propanol, the obtained product was used in the next step.

### Example 2

#### Preparation of 2-chloromethyl-4-(3- methoxy-propoxy)-3-methyl pyridine

50g 4-(3-methoxy-propoxy)-2,3-dimethyl-pyridine N-oxide, 150ml acetic anhydride and 8 drops of concentrated sulfuric acid were put into a reaction flask and reacted at 90 °Cfor 3 hours in oil bath. The acetic anhydride was vaporized under vacuum. 100ml water and 20g sodium hydroxide at ambient temperature were added to the flask and reacted at 50 °C for 1 hour in water bath. And then the reaction solution was cooled, extracted by chloroform, dried, filtered and concentrated till dry. 200ml fresh chloroform and 30ml thionyl chloride were added to the residue and the reaction took place at ambient temperature for 5 hours and the reaction solution was concentrated under vacuum. After that, 400ml water was added to the residue and the pH value was adjusted to 8 with sodium carbonate solution. Finally, the expected product (brown semi-solid) was produced after the procedures of extracting the reaction solution by chloroform, drying and concentration.

### Example 3

#### Preparation of 2-{[4- (3- methoxy-propoxy)-3-methyl pyridine -2-yl]-methylthio}-1H-5-difluoromethoxyl-benzimidazole

42g 2-chloromethyl-4-(3-methoxy-propoxy)-3-methyl pyridine, 29g 5-difluoromethoxyl-2-mercapto benzimidazole, 0.3g tetrabutyl ammonium bromide, 35g potassium carbonate and 200ml anhydrous ethanol were put into a reaction flask and reacted at 70 °C for one hour. After filtration and concentration, the residue was mixed with 300ml dichloromethane, water-washed, dried, filtered, concentrated till dry, and then mixed with 100ml ethyl acetate for recrystallization, thereby a solid product was obtained.

**Example 4**

**Preparation of 2-{[4-3-methoxy-propoxy)-3-methyl pyridine-2-yl]-methylthio}-1H-6-methoxypyridoimidazole**

42g 2-chloromethyl-4-(3-methoxy-propoxy)-3-methyl pyridine, 27g 6-methoxyl-2-mercapto pyridoimidazole, 0.3g tetrabutyl ammonium bromide, 35g potassium carbonate and 200ml anhydrous ethanol were put into a reaction flask and reacted at 70 °C for 1 hour. After filtration and concentration, the residue was mixed with 300ml dichloromethane and then water-washed, dried, filtered and then concentrated till dry, and recrystallized in 100ml ethyl acetate to obtain a solid product.

**Example 5**

**Preparation of 2-{[4-3-methoxy-propoxy)-3-methylpyridine-2-yl]-methyl-sulfinyl}-1H-5-difluoromethoxy benzimidazole sodium salt**

4.4g (10.65mmol) 2-{[4-3-methoxy-propoxy)-3-methyl pyridine-2-yl]-methyl-sulfinyl]--1H-5-difluoromethoxyl-benzimidazole was added to a solution prepared with 0.412g (10.3mmol) sodium hydroxide and 100ml water. After dissolving, 200ml ethanol was put into the solution which was then concentrated till dry under vacuum. The resulting residue was mixed with tert-butyl methyl ether and then filtered, dried and finally the expected product was obtained.

**Example 6**

**Preparation of 2-{[4-(3-methoxy-propoxy)-3-methylpyridine-2-yl]-methyl-sulfonyl}-1H-5-difluoromethoxy benzimidazole lithium salt**

4.4g(10.65mmol) 2-{[4-(3-methoxy-propoxy)-3-methylpyridine-2-yl]-methyl-sulfonyl]-1H-5-difluoromethoxyl-benzimidazole was added to the solution prepared with 0.25g (10.3mmol) lithium hydroxide and 100ml water. After dissolving, 200ml ethanol was put into the solution which was then concentrated till dry under vacuum. The residue was mixed with tert-butyl methyl ether and then filtered and dried to obtain the product.

**Example 7**

**Preparation of 2-{[4-{3-methoxy-propoxy)-3-methylpyridine-2-yl]-methyl-sulfinyl}-1H-5- difluoromethoxy benzimidazole calcium salt**

0.735g (0.005mol) calcium chloride dihydrate was dissolved in 6ml water, then the solution was dropped slowly into 4.35g (0.01 mol) of 2-{ [4-(3-methoxy-propoxy)-3-methylpyridine-2-yl]-methyl-sulfinyl}-1H-5-difluoromethoxy benzimidazole sodium salt (prepared in Example 5) in 25ml water. Then stirred magnetically for 1 hour, and then the reaction solution was filtered, the obtained filter cake was washed by water and vacuum dried at 40 °C for 10 hours to obtain the title product.

**Example 8**

**Preparation for 2-{[4-3-methoxy-propox)-3-methyl pyridine-2-yl]-Methyl-sulfinyl}-1H-6-methoxy-pyridoimidazole sodium salt**

The preparation method is similar to that used in Example 5 except that 4.4g (10.65mmol) 2- {[4-(3-methoxy-propoxy)-3-methylpyridine-2-YL]-methyl-sulfinyl}-1H-5-difluoromethoxyl-benzimidazole was replaced by 4.0g (10.65mmol) 2-{[4- (3- methoxy-propoxy)-3-methyl pyridine -2-yl]-methyl sulfinyl}-1H-6-methoxy-pyridoimidazole.

**Example 9**

**Preparation of 2-{[4-(3-methoxy-propoxy)-3-methylpyridine-2-yl]-methyl-sulfonyl}-1H-6-methoxy pyridoimidazole calcium salt**

The preparation method is similar to that used in Example 7 except that 4.35g (0.01mol) 2- [4-(3-methoxy-propoxy)-3-methyl pyridine-2-YL]-methyl-sulfonyl}-1H-5-difluoromethoxyl-benzimidazole sodium salt prepared in Example 5 was replaced by 4.0g (0.01mol) 2-{[4-(-methoxy-propox)-3-methylpyridine-2-yl]-methyl-sulfonyl}-1H-6-methoxy

pyridoimidazole sodium salt prepared in Example 8.

**Example 10**

**Preparation of 2-{[4-(3-methoxy-propoxy)-3-methyl pyridine-2-yl]-methyl-sulfonyl}-1H-6-methoxy pyridoimidazole magnesium salt**

10.1 g (0.05mol) magnesium chloride hexahydrate was dissolved in 100ml water, and 10ml of the obtained solution was dropped slowly into 4.0g (0.01mol) 2-{[4-(3-methoxy-propoxy)-3-methyl pyridine-2-yl]-methyl-sulfonyl}-1H-6- methoxy pyridoimidazole sodium salt prepared in Example 8 in 15ml water. After stirring, filtration and vacuum drying, the expected product was thus obtained.

**Example 11**

**Preparation of (S)-2-{[4- (3-methoxy-propoxy)-3- methyl pyridine -2-yl]-methyl-sulfinyl}-1H-5-difluoromethoxy benzimidazole**

7.8g 2-{[4- (3- methoxy-propoxy)-3- methyl pyridine -2-yl]- methylthio }-1H-5-difluoromethoxy benzimidazole and 32ml toluene were put into a reaction flask and heated to 54°C with agitation, and then added with 2.8ml (-)-diisopropyl-D-tartaric acid, 2.2ml isopropyl-titanium peroxide and 56μl distilled water. After that, the reaction took place for 50 minutes at a constant temperature. 1.25ml N, N-isopropyl ethamine was added to the reaction when the temperature dropped to 30°C and then added with 4.6ml cumene hydroperoxide in drops, and the reaction took place at 30°C for 1 hour. The reaction solution was extracted with 12.5% ammonia (3x25ml), collecting all three extraction solutions, adjusted to pH=7 with acetic acid, and finally extracted with 4-methyl-2-pentanone (3×12ml). The extraction solution was dried, filtered, concentrated till dry, and recrystillized with ethyl acetate and n-hexane to obtained the title compound (5.2g).

**Experiment 1**

**Effect on gastric acid secretion in rats**

Note: In Compound (II) of the invention, $R_2$ is methyl and $R_3$ is hydrogen.

**1. Purpose of the experiment**

To compare effects of prazole compounds on gastric acid secretion

**2. Materials**

2.1 Animal

Wistar rat, weight 180-220g, male, obtained from Institute of Zoology, Chinese Academy of Medical Sciences.

2.2 Drugs for administration

Sodium rabeprazole, compound (II) of the present invention and tenatoprazole, provided by Jiangsu Hansen Pharmaceutical Co., Ltd.

2.3 Preparation of the drug

Added 1 drop of Tween-80 as a solubilizing assistant and prepared the required concentrations with distilled water.

**3. Methods and Results**

**Methods:** 48-hour fasting rats were divided into groups randomly. Pylon ligation was carried out under ether anaesthesia. During the operation, different doses of the drugs were administered respectively through the duodenum and the volumn for administration is 5ml/kg. The rats were sacrificed at 5 hours after the operation and the gastric juice was collected and measured. The content of HCl in gastric juice is determined by titration with 0.1mol/L NaOH and the

significance test was made by t-programme. Results: See the table below. It has been reported in a number of literatures that over strong effect of inhibition of a prazole drug on gastric acid secretion may induce a feed-back mechanism of gastric sinus, resulting in a high level of gastric secretin in plasma and the formation of gastric cancer due to proliferation of secretory cell. In contrast, if this drug has a weak effect of inhibition on gastric acid secretion, there will be no a feed-back mechanism of gastric sinus induced, or a high gastric secretion in plasma thus caused and accordingly, it is impossible to make a proliferation of secretory cell hyperplasia and the risk of gastric cancer will be greatly decreased. The effect of inhibition of compound (II) of the present invention on gastric acid secretion is weak and thus may reduce the risk of gastric cancer.

**Table 1. Effect of prazole compounds on gastric acid secretion model induced by pylorus ligation in rats**

| Group | Dose μmol/kg (mg/kg) | No. of rats | Amount of gastric acid mmol/stomach | Amount of gastric juice ml/stomach |
|---|---|---|---|---|
| Control animal | --- | 10 | 0.28±0.09 | 3.7±0.8 |
| Sodium Rabeprazole | 20.0(7.62) | 10 | 0.17±0.08* | 3.7±0.8 |
| Sodium Rabeprazole | 6.7(2.53) | 10 | 0.25±0.10 | 4.0±0.7 |
| Compound (II) | 20.0(7.72) | 10 | 0.26±0.10 | 4.0±1.2 |
| Compound (II) | 6.7(2.57) | 10 | 0.25±0.12 | 3.7±1.0 |
| Tenatoprazole | 20.0(6.92) | 10 | 0.14±0.03*** | 2.8±0.5* |
| Tenatoprazole | 6.7(2.31) | 10 | 0.16±0.06** | 2.8±0.7 |

Compared with the control group: *p<0.05, **p<0.01, ***p<0.001.

**Experiment 2**

**Effect on gastric ulcer model induced by anhydrous ethanol in rats**

Note: In compound (I), $R_1$ is difluoromethyl and $R_3$ is sodium. In compound (II), $R_2$ is methyl and $R_3$ is sodium.

**1. Purpose of the experiment**

To understand the effects of Compound (I), Compound (II) and Sodium Rabeprazole on anti-ulcer.

**2. Materials**

2.1 Animal

Wistar rat, weight: 180-220g, male, obtained from Institute of Zoology, Chinese Academy of Medical Sciences.

2.2 Drug

Compound (I) and Compound (II) of the present invention and sodium rabeprazole, provided by Jiangsu Hansen Pharmaceutical Co., Ltd.

2.3 Preparation of the Drug

The required concentration is prepared with distilled water and the volumn for administration of drug is 10ml/kg.

**3. Methods and Results**

Methods: 40-hour fasting rats were randomly divided into groups and treated with different doses of sodium rabeprazole, compound (I), compound (II) and distilled water (as control) by gastric perfusion, respectively. After 0.5 hour, each rat was administered with 1ml anhydrous ethanol and then rats were sacrificed 1 hour later. The stomachs were taken out and injected with 10ml fix solution. After fixed in 3% formaldehyde solution, the stomachs were dissected and the ulcer areas were measured. The significance test was made by t-programme.
**Results:** The ulcer areas of rats administered with sodium rabeprazole were reduced. Compounds (I) & (II) have same function and even have a more significant effect. See Table 1 for details.

**Table 1: Protection of sodium rabeprazole, compounds (I) & (II) on gastric ulcer model induced by anhydrous ethanol in rats**

| Group | Dose (mg/kg) | No. of rats | Ulcer area | P≤ |
|---|---|---|---|---|
| Control Group | | 8 | 24.8±12.4 | |
| Sodium Rabeprazole | 10 | 5 | 11.1±2.7 | 0.05 |
| Sodium Rabeprazole | 3 | 6 | 26.4±20.6 | |
| Compound (I) | 10 | 5 | 5.5±9.9 | 0.05 |
| Compound (I) | 3 | 6 | 12.1±18.1 | |
| Compound (II) | 10 | 5 | 3.2±4.5 | 0.01 |
| Compound (II) | 3 | 6 | 6.6±5.8 | 0.01 |

**Experiment 3**

**Effect on chronic gastric ulcer model induced by acetylsalicylic acid in rats**

In this example, compound (II) of the present invention was used, wherein, $R_2$ is methyl and $R_3$ is hydrogen.
**Methods:** Abdominal cavities of fasting rats were cut under the anaesthesia of sodium pentobarbital and gently stretched the stomachs. The glandular stomachs near pylons were injected with 20μl 30% acetic acid and then the abdominal cavities were re-closed. At the day the operation finished, rats were randomly divided into groups and started to be administered with drugs of 10ml/kg once a day for 10 continuous days. Rats were then sacrificed in the following day when the last drug was administered, and stomachs were taken out and were injected with 10ml water, and then were put into 3% formaldehyde solution to be fixed for 30 minutes. The stomachs were dissected along their greater curvatures and stretched on a glass plate and the ulcer areas were measured. The significance test was made by t-programme.
Results: Rats with the treatment of prazole compounds showed smaller ulcer areas than those of the control group. Specifically, a significant difference was indicated between the following groups and control group: large dosage group of sodium rabeprazole, large dosage group and small dosage group of Compound (II), and large dosage group of tenatoprazole. See the table below for details.

**Protection of prazole compounds on gastric ulcer model induced by acetic acid in rats**

| Group | Dose μmol/kg(mg/kg) | No. of Rats | Ulcer area $mm^2$ $x \pm s$ |
|---|---|---|---|
| Control group | --- | 12 | 9.4±4.1 |
| Sodium Rabeprazole | 60(22.86) | 12 | 4.8±3.1** |
| Sodium Rabeprazole | 20(7.62) | 12 | 6.2±3.5 |
| Compound (II) | 60(23.16) | 12 | 5.5±4.1 * |
| Compound (II) | 20(7.72) | 12 | 4.9±2.8** |
| Tenatoprazole | 60(20.76) | 12 | 5.8±3.6* |
| Tenatoprazole | 20(6.92) | 12 | 7.6±3.6 |

Compared with the control group: *p<0.05, **p<0.01.
Compound (II) 2 of the present invention.

**Experiment 4**

**Comparative study of the effect on gastric ulcer model induced by indomethacin in rats**

Note: In compound (II) of the present invention, $R_2$ is methyl and $R_3$ is hydrogen.

### 1. Purpose of the Experiment

To learn the effects on ulcer rat models induced by indomethacin, pylon ligation, cold water restraint stress and acetic acid, and to observe effects of compound (II) on gastric acid secretion and pepsin activity as well as the effect of Prazole B on $H^+$-$K^+$-ATPase in gastric parietal cell in rats.

### 2 . Experiment Materials

2.1 Animal

Wistar rats, weight: 180-220g, same number of male and female, provided by Institute of Zoology, Chinese Academy of Medical Sciences. (Certification of Qualification of Animal: scxk Beijing 2000-0006)

2.2 Drugs

Compound (II): provided by Jiangsu Hansen Pharmaceutical Co., Ltd
Sodium Rabeprazole: provided by Jiangsu Hansen Pharmaceutical Co., Ltd

2.3 Preparation of the drugs

0.5% methylcellulose sodium: 2g/L $NaHCO_3$ is contained and pH value is adjusted to 9.0 with NaOH.
Preparation: The drug was porphyrized and diluted with 0.5% methylcellulose sodium to the required concentration. It must be used up in 30 minutes.

### 3. Effects on gastric ulcer model induced by indomethacin in rats

**Methods:** Sixty 48-hour fasting rats were divided into six groups and gastric-perfused with different doses of compound (II), sodium rabeprazole and 0.5% methylcellulose sodium (as control), respectively. After 0.5 hour, 20mg/kg indomethacin was injected into the abdominal cavities. Six hours later, rats were sacrificed by cervical dislocation and the stomachs were taken out. After injected with 10ml water, stomachs were then fixed in 3% formaldehyde solution. The hemorrhagic spots were counted. A significance test was carried out by t-programme.
Results: see Table 5 for details. It was indicated that after the treatment of compound (II) by gastric perfusion, the hemorrhagic spots on gastric mucosal induced by indomethacin in rats were remarkably reduced. It also showed a dose dependent effect. Ulcer inhibition of $ED_{50}$ was 14.1 μmol/kg. It thus proved that compound (II) has an obvious function of protection of gastric mucosal hemorrhage induce by indomethacin. Sodium Rabeprazole has the same function but the effect is lower than that of compound (II).

**Effect of compound (II) on ulcer model induced by indomethacin (n=10) in rats**

| Group | Dose μmol/kg | No. of ulcers x ± s | Inhibition rate % |
|---|---|---|---|
| Control Group | --- | 11.2±7.2 | |
| | 5 | 10.8±8.7 | 3.6 |
| Compound (II) | 10 | 8.4±6.1 | 25.0 |
| | 20 | 2.9±4.1** | 74.1 |
| | 40 | 0.3±0.6*** | 97.3 |
| Sodium Rabeprazole | 40 | 3.1±4.6** | 72.3 |

Ulcer inhibition $ED_{50}$=14.1 μmol/kg (95% confidence limit: 12.9-15.5).
Compared with the control group **p<0.01, ***p<0.001.

### Experiment 5

### Effect on ulcer model induced by pylori ligation in rats

In Compound (II) used in this example, $R_2$ is methyl and $R_3$ is hydrogen.
Methods: Sixty 40 hour-fasting rats were divided into 6 groups according to Table 2. A pylori ligation operation was carried out under the anaesthesia of ether. Different doses of compound (II), sodium rabeprazole and 0.5% methylcellulose sodium (as control) were administered through duodenum during the operation, respectively. Rats were sacrificed

at 18 hours after the operation and the stomachs were taken out and then fixed in 3% formaldehyde solution after injecting 10ml water. The stomachs were dissected and the number of gastric ulcers was counted. The result was tested by a significance test of t-promgramme.
Results: see the table below for details. It was shown that after treated with compound (II), ulcers in the rats were obviously mitigated and the inhibition rate was 41.4~96.7%. It also showed a dose dependent effect. The ulcer inhibition of $ED_{50}$ was 16μmol/kg. It is thus obvious that compound (II) has a significant function of protection of gastric ulcer model induced by pylori ligation. Sodium rabeprazole has an essentially same function.

**Effect of compound (II) on ulcer rat models induced by pylori ligation (n=10)**

| Group | Dose μmol/kg | No. of ulcers x ± s | Inhibition Rate % |
|---|---|---|---|
| Control Group | --- | 24.2±15.7 | |
| Compound (II) | 10 | 14.2±12.0 | 41.4 |
| | 20 | 11.9±9.3* | 50.9 |
| | 40 | 6.7±8.7** | 72.3 |
| | 80 | 0.8±1.8*** | 96.7 |
| Sodium Rabeprazole | 40 | 8.9±14.7** | 63.2 |

Ulcer inhibition of $ED_{50}$=16.0μ mol/kg (95% confidence limit: 12.9-18.9)。
Compared with the control group *p<0.05, **p<0.01, ***p<0.001。

**Experiment 6**

**Studies on in vivo pharmacokinetics of rabeprazole, tenatoprazole and compound (II) in Beagle dogs**

This example used compound (II) of the present invention, wherein $R_2$ is methyl and $R_3$ is hydrogen.
**Methods:** Six healthy male beagle dogs with weight 5.5~6.7kg were randomly divided into three groups for triple cross over administration. They were perfused with rabeprazole, tenatoprazole and compound (II) respectively after one-night fasting. The dose of each drug was 5mg/kg. Each dog was fed with 50ml water by gastric perfusion after administration of the drug. 2ml blood was taken respectively from forelimb saphenous vein at 0.25, 0.5, 1, 2, 3, 4, 5, 6, 7, 8, 10, 12 hours after tenatoprazole was administered, at 5, 10, 20, 30, 40, 50, 60, 75, 90, 120, 180 minutes after rabeprazole was administered, and at 1, 2, 3, 3.5, 4, 4.5, 5, 6, 7, 8, 10, 12 hours after compound (II) was administered, and then put each blood sample in each test tube treated with EDTA, then centrifuging for 10 min at 3500rpm. The upper plasma was removed from the test tube into another test tube containing 50μl 2%NaOH solution and reserved at -20°C. The plasma drug concentration was determined by HPLC. Cross-over administration to dogs was performed one week later.

**Tabel 1: Grouping of dogs for test and administration by gastric perfusion**

| Dog | Sex | Weight (kg) | Order of administration |
|---|---|---|---|
| A | Male | 6.0 | rabeprazole / compound (II)/ tenatoprazole |
| B | Male | 5.5 | rabeprazole / compound (II)/ tenatoprazole |
| C | Male | 6.0 | compound (II)/ tenatoprazole / rabeprazole |
| D | Male | 5.6 | compound (II)/ tenatoprazole / rabeprazole |
| E | Male | 6.2 | tenatoprazole /rabeprazol / compound (II) |
| F | Male | 6.3 | tenatoprazole /rabeprazol / compound (II) |

The calculation method for pharmacokinetic parameters: compartment model is estimated by software DAS1.0. The calculation of the related pharmacokinetic parameters by moment method, i.e., area under the curve (AUC), mean residence time (MRT) and clearance (CI/F), can be carried out respectively according to each of the following equations:

$$AUC^{24}=\Sigma(C_i+C_{i-1})(t_i - t_{i-1})/2$$

$$AUC=AUC^{24}+C_{24}/\lambda_Z$$

$$AUMC^{24}=\Sigma(C_i \cdot t_i+C_{i-1}\cdot t_{i-1})(t_i - t_{i-1})/2$$

$$AUMC=AUMC^{24}+C_{24}(24/\lambda_z+1/\lambda^2_Z)$$

$$MRT=AUMC/AUC$$

$$Cl/F=D/AUC$$

Wherein C24 is the blood drug concentration at 12 hours after administration, $\lambda z$ is the terminal phase rate constant, obtained by linear regression of terminal phase ln C~t. T1/2=0.693/$\lambda z$, Tmax and Cmaxare are measured values.

**Results**: Data of blood drug concentration-time: Concentrations of rabeprazole, tenatoprazole and compound (II) in plasma after 6 dogs were administrated with 5mg/kg each of them respectively can be seen in Tables 1-2 and Figures 1-2.

**Table 2. Data of plasma concentration of rabeprazole-time after rabeprazole was administered to 6 dogs at 5mg/kg(μg/ml)**

| Dog | 5 | 10 | 20 | 30 | 40 | 50 | 60 | 75 | 90 | 120 | 180min |
|---|---|---|---|---|---|---|---|---|---|---|---|
| A | 0.00 | 0.00 | 0.00 | 0.62 | 0.25 | 0.00 | 0.19 | 0.00 | 0.00 | 0.00 | 0.00 |
| B | 0.00 | 0.00 | 0.00 | 1.35 | 0.58 | 0.00 | 0.23 | 0.17 | 0.13 | 0.00 | 0.00 |
| C | 0.00 | 0.00 | 0.00 | 0.00 | 0.00 | 0.00 | 0.00 | 0.00 | 0.00 | 0.00 | 0.00 |
| D | 0.00 | 0.00 | 0.00 | 0.00 | 0.00 | 0.00 | 0.00 | 0.00 | 0.00 | 0.00 | 0.00 |
| E | 0.00 | 0.00 | 1.41 | 1.57 | 0.43 | 0.40 | 0.16 | 0.03 | 0.00 | 0.00 | 0.00 |
| F | 0.00 | 0.00 | 0.30 | 0.64 | 0.60 | 0.32 | 0.23 | 0.00 | 0.00 | 0.00 | 0.00 |
| $\bar{x}$ | 0.00 | 0.00 | 0.29 | 0.70 | 0.31 | 0.12 | 0.14 | 0.03 | 0.02 | 0.00 | 0.00 |
| s | 0.00 | 0.00 | 0.56 | 0.66 | 0.27 | 0.19 | 0.11 | 0.07 | 0.05 | 0.00 | 0.00 |

**Table 3. Data of plasma concentration of tenatoprazole-time after tenatoprazole was administered to 6 dogs at 5mg/kg (μg/ml)**

| Dog | 0.25 | 0.50 | 1 | 2 | 3 | 4 | 5 | 6 | 7 | 8 | 10 | 12h |
|---|---|---|---|---|---|---|---|---|---|---|---|---|
| A | 0.00 | 0.00 | 0.00 | 0.67 | 3.96 | 4.44 | 3.61 | 2.17 | 1.47 | 0.78 | 0.27 | 0.07 |
| B | 1.71 | 3.83 | 4.44 | 2.74 | 1.74 | 1.51 | 0.92 | 0.70 | 0.42 | 0.36 | 0.16 | 0.10 |
| C | 0.00 | 0.00 | 0.00 | 0.87 | 6.48 | 5.23 | 3.53 | 2.56 | 2.03 | 1.34 | 0.59 | 0.22 |
| D | 0.03 | 0.32 | 1.24 | 1.67 | 3.32 | 1.59 | 1.57 | 1.59 | 1.34 | 1.28 | 0.69 | 0.32 |
| E | 0.00 | 1.71 | 3.52 | 3.18 | 2.29 | 1.62 | 1.13 | 0.70 | 0.49 | 0.34 | 0.22 | 0.13 |
| F | 0.00 | 1.71 | 3.48 | 2.67 | 1.97 | 1.45 | 1.36 | 0.93 | 0.81 | 0.56 | 0.33 | 0.14 |
| $\bar{x}$ | 0.29 | 1.26 | 2.11 | 1.97 | 3.29 | 2.64 | 2.02 | 1.44 | 1.10 | 0.78 | 0.38 | 0.16 |
| s | 0.69 | 1.49 | 1.95 | 1.05 | 1.77 | 1.72 | 1.22 | 0.79 | 0.63 | 0.44 | 0.21 | 0.09 |

**Table 4. Data of plasma concentration of compound (II) -time after compound (II) was administered to 6 dogs at 5mg/kg (μg/ml)**

| Dog | 1 | 2 | 3 | 3.5 | 4 | 4.5 | 5 | 6 | 7 | 8 | 10 | 12h |
|---|---|---|---|---|---|---|---|---|---|---|---|---|
| A | 0.00 | 0.00 | 7.89 | 11.19 | 8.26 | 7.94 | 6.82 | 6.50 | 3.09 | 3.03 | 1.25 | 0.45 |
| B | 2.37 | 2.50 | 1.43 | 1.81 | 1.67 | 1.25 | 1.09 | 0.85 | 0.55 | 0.28 | 0.10 | 0.10 |
| C | 0.00 | 0.57 | 5.49 | 6.73 | 5.39 | 4.92 | 4.33 | 3.19 | 1.75 | 0.71 | 0.12 | 0.00 |
| D | 0.66 | 0.71 | 3.39 | 4.66 | 4.81 | 4.99 | 4.61 | 3.39 | 1.36 | 0.58 | 0.09 | 0.00 |
| E | 5.26 | 6.86 | 6.19 | 5.16 | 4.36 | 3.63 | 3.19 | 2.10 | 1.55 | 0.95 | 0.33 | 0.24 |
| F | 2.60 | 3.13 | 3.40 | 3.17 | 2.71 | 2.37 | 1.89 | 1.49 | 1.05 | 0.59 | 0.34 | 0.22 |
| $\bar{x}$ | 1.82 | 2.29 | 4.63 | 5.45 | 4.53 | 4.18 | 3.66 | 2.92 | 1.56 | 1.02 | 0.37 | 0.17 |
| s | 2.04 | 2.54 | 2.32 | 3.28 | 2.29 | 2.35 | 2.06 | 2.01 | 0.86 | 1.01 | 0.44 | 0.17 |
| a, If the concentration is lower than the minimum detection concentration, it can be considered as "0'. | | | | | | | | | | | | |

It can be seen from the above data that concentrations of rabeprazole are mostly below the detectable limit, i.e., the plasma concentrations of rabeprazole in dogs are low (also see Figs.1A-F and Fig.2), and the peak value is only 1.57μg/ml. Additionally, rabeprazole is eliminated quickly, and half life of rabeprazole is less than half hour. The pharmacokinetic behaviors of tenatoprazole and compound (II) are similar and therefore, the comparison will be conducted only between these two drugs as shown below.

**Pharmacokinetic parameters:** The pharmacokinetic parameters after administrating 5mg/kg of each of tenatoprazole and compound (II) to Beagle dogs were estimated by Moment method as shown in Table 5 and Table 6:

**Table 5. Pharmacokinetic parameters after orally administrating 5mg/kg tenatoprazole to Beagle dogs**

| No. | $T_{max}$ h | $T_{1/2}$ h | MRT h | $C_{max}$ μg/ml | $AUC^{12}$ μg.h/ml | AUC μg.h/ml |
|---|---|---|---|---|---|---|
| A | 4.0 | 3.43 | 4.96 | 4.44 | 18.44 | 18.70 |
| B | 1.0 | 3.68 | 5.31 | 4.44 | 14.18 | 15.75 |
| C | 3.0 | 3.58 | 5.17 | 6.48 | 24.54 | 25.18 |
| D | 3.0 | 4.21 | 6.08 | 3.32 | 15.79 | 17.27 |
| E | 1.0 | 4.18 | 6.04 | 3.52 | 13.99 | 15.86 |
| F | 1.0 | 3.37 | 4.86 | 3.48 | 14.30 | 15.39 |
| $\bar{x}$ | 2.17 | 3.74 | 5.40 | 4.28 | 16.87 | 18.02 |
| s | 1.33 | 0.37 | 0.53 | 1.18 | 4.11 | 3.71 |

**Table 6: Pharmacokinetic parameters after orally administrating 5mg/kg compound (II) to Beagle dogs**

| No. | $T_{max}$ h | $T_{1/2}$ h | MRT h | $C_{max}$ μg/ml | $AUC^{12}$ μg·h/ml | AUC μg.h/ml |
|---|---|---|---|---|---|---|
| A | 3.5 | 3.56 | 5.13 | 11.19 | 49.70 | 50.47 |
| B | 2.0 | 3.80 | 5.49 | 2.50 | 11.26 | 12.46 |
| C | 3.5 | 3.22 | 4.64 | 6.73 | 22.87 | 23.04 |
| D | 4.5 | 3.22 | 4.65 | 4.99 | 20.32 | 20.44 |
| E | 2.0 | 2.77 | 3.99 | 6.86 | 31.71 | 32.66 |
| F | 3.0 | 3.50 | 5.05 | 3.40 | 18.15 | 19.56 |
| $\bar{x}$ | 3.1 | 3.34 | 4.83 | 5.95 | 25.67 | 26.44 |
| s | 1.0 | 0.36 | 0.52 | 3.11 | 13.52 | 13.46 |

**The comparison of pharmacokinetic parameters between tenatoprazole and compound (II) in Beagle dogs**

Table 7 shows results of the main pharmacokinetic parameters after orally administrating 5mg/kg of tenatoprazole and compound (II) respectively in Beagle dogs

**Table 7-1. Comparisons of peak concentrations between tenatoprazole and Compound (II) after orally administered to Beagle dogs at 5mg/kg respectively**

| No. | Tenatoprazole | | Compound (II) | |
|---|---|---|---|---|
| | $C_{max}$ | $Ln(C_{max})$ | $C_{max}$ | $Ln(C_{max})$ |
| A | 4.44 | 1.49 | 11.19 | 2.42 |
| B | 4.44 | 1.49 | 2.50 | 0.92 |
| C | 6.48 | 1.87 | 6.73 | 1.91 |
| D | 3.32 | 1.20 | 4.99 | 1.61 |
| E | 3.52 | 1.26 | 6.86 | 1.93 |
| F | 3.48 | 1.25 | 3.40 | 1.22 |
| $\bar{x}$ | 4.28 | 1.43 | 5.95 | 1.67 |
| s | 1.18 | 0.25 | 3.11 | 0.54 |

**Table 7-2. Comparisons of AUC12 between tenatoprazole and compound (II) after orally administered to Beagle dogs at 5mg/kg respectively**

| | Tenato-trazole | | Compound (II) | |
|---|---|---|---|---|
| | $AUC^{12}$ | $Ln(AUC^{12})$ | $AUC^{12}$ | $Ln(AUC^{12})$ |
| A | 18.44 | 2.91 | 49.70 | 3.91 |
| B | 14.18 | 2.65 | 11.26 | 2.42 |
| C | 24.54 | 3.20 | 22.87 | 3.13 |
| D | 15.79 | 2.76 | 20.32 | 3.01 |
| E | 13.99 | 2.64 | 31.71 | 3.46 |
| F | 14.30 | 2.66 | 18.15 | 2.90 |
| $\bar{x}$ | 16.87 | 2.80 | 25.67 | 3.14 |
| s | 4.11 | 0.22 | 13.52 | 0.51 |

**Table 7-3. Comparisons of AUC between tenatoprazole and compound (II) after orally administered to Beagle dogs at 5mg/kg respectively**

| | Tenatoprazole | | Compound (II) | |
|---|---|---|---|---|
| | AUC | Ln(AUC) | AUC | Ln(AUC) |
| A | 18.70 | 2.93 | 50.47 | 3.92 |
| B | 15.75 | 2.76 | 12.46 | 2.52 |
| C | 25.18 | 3.23 | 23.04 | 3.14 |
| D | 17.27 | 2.85 | 20.44 | 3.02 |
| E | 15.86 | 2.76 | 32.66 | 3.49 |
| F | 15.39 | 2.73 | 19.56 | 2.97 |
| $\bar{x}$ | 18.02 | 2.88 | 26.44 | 3.18 |
| s | 3.71 | 0.19 | 13.46 | 0.48 |

**Table 7-4. Comparisons of peak time, half life and MRT between tenatoprazole and compound (II) after orally administered to Beagle dogs at 5mg/kg respectively**

| | $T_{max}$ | | $T_{1/2}$ | | MRT | |
|---|---|---|---|---|---|---|
| | Tenatoprazole | Compound (II) | Tenatoprazole | Compound (II) | Tenatoprazole | Compound (II) |
| A | 4.0 | 3.5 | 3.43 | 3.56 | 4.96 | 5.13 |
| B | 1.0 | 2.0 | 3.68 | 3.80 | 5.31 | 5.49 |
| C | 3.0 | 3.5 | 3.58 | 3.22 | 5.17 | 4.64 |
| D | 3.0 | 4.5 | 4.21 | 3.22 | 6.08 | 4.65 |
| E | 1.0 | 2.0 | 4.18 | 2.77 | 6.04 | 3.99 |
| F | 1.0 | 3.0 | 3.37 | 3.50 | 4.86 | 5.05 |
| $\bar{x}$ | 2.17 | 3.1 | 3.74 | 3.34 | 5.40 | 4.83 |
| s | 1.33 | 1.0 | 0.37 | 0.36 | 0.53 | 0.52 |

It can be seen from the above results that after administrating 5mg/kg of tenatoprazole and compound (II) to Beagl dogs respectively, the pharmacokinetic parameters are: Cmax: tenatoprazole is 4.28+1.18, compound (II) is 5.95+3.11; Tmax: tenatoprazole is 2.17+1.33, compound (II) is 3.1+1.0; T1/2: tenatoprazole is 3.74+0.37, compound (II) is 3.34+0.36; MRT: tenatoprazole is 5.40+0.53, compound (II) is 4.83+0.52; AUC12: tenatoprazole is 16.87+4.11, compound (II) is 25.67+13.52; AUC: tenatoprazole is 18.02+3.71, compound (II) is 26.44+13.46.

**Conclusion:** It can be concluded that the pharmacokinetic behaviors of compound (II) are remarkably improved compared with those of rabeprazole. In particular, compound (II) shows a better absorption and longer elimination half-life. As for the half life, tenatoprazole and compound (II) are similar, but compound (II) shows an increase with respect to Cmax and AUC, which indicates a much better absorption for compound (II).

**Experiment 7**

Preparation

| | | |
|---|---|---|
| formulation: | Compound (II) | 10g |
| | Starch | 30g |
| | Sucrose | 20g |
| | Microcrystalline cellulose | 10g |
| | 0.5% CMC solution | Proper amount |
| | Magnesium stearate | 0.7g |
| | | 1000 tablets |

The grain-making by conventional wet process, tablet pressing and enteric coating are carried out.

## Claims

**1.** . A derivative of the compound of Formula (I) or (II) or a salt thereof

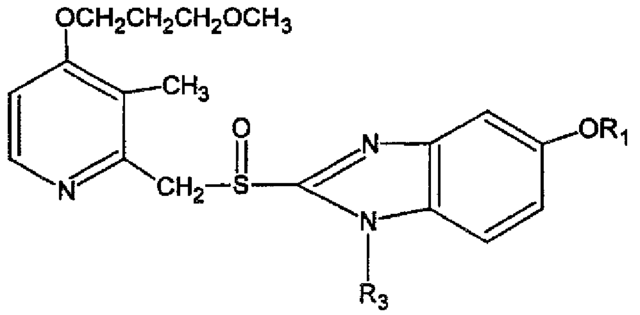

(I)

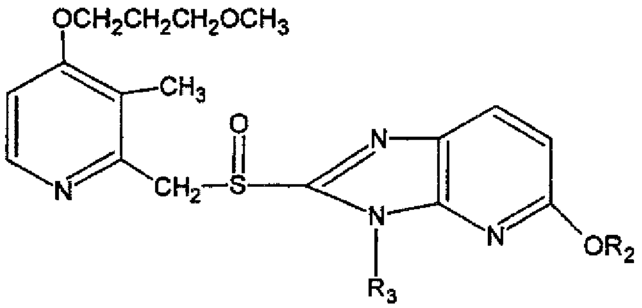

(II)

Wherein $R_1$ represents lower alkyl or halogen substituted lower alkyl, $R_2$ represents straight-chain or branched-chain alkyl having 1 to 4 carbon atoms, and $R_3$ represents hydrogen, alkali metal such as lithium, sodium or potassium, or alkaline-earth metal such as magnesium or calcium.

2. The derivative or salt thereof of Claim 1, **characterized in that** $R_1$ represents methyl or difluoromethyl and $R_3$ represents hydrogen or sodium in formula (I).

3. The derivative or salt thereof of Claim 1, **characterized in that** the salt represented by formula (I) or (II) is a hemi-magnesium salt or a hemi-calcium salt when $R_3$ represents an alkali earth metal such as magnesium or calcium.

4. The derivative or salt thereof of Claim 1, **characterized in that** $R_1$ represents difluoromethyl in Formula (I).

5. The derivative or salt thereof of Claim 1, **characterized in that** $R_2$ represents methyl and $R_3$ represents hydrogen or sodium in formula (II).

6. The derivative or salt thereof of Claim 1, **characterized in that** said derivative or salt thereof is a compound or a salt thereof in the form of single (R) or (S) enantiomer, or rich in an enantiomer.

7. A pharmaceutical composition comprising a derivative and/or a pharmaceutically acceptable salt thereof of any one of claims 1-6.

8. Use of a derivative and/or a pharmaceutically acceptable salt thereof of any one of Claims 1 to 6 in preparation of a medicament for killing *Helicobacter pylori.*

9. Use of a derivative and/or a pharmaceutically acceptable salt thereof of any one of Claims 1 to 6 in preparation of a medicament for treating and/or preventing gastric and/or intestinal diseases.

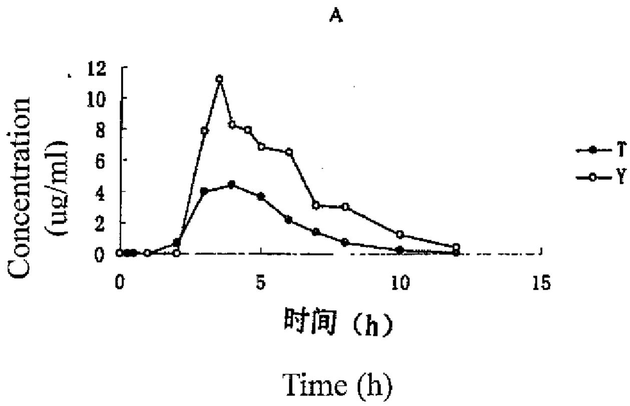


FIG. 1A

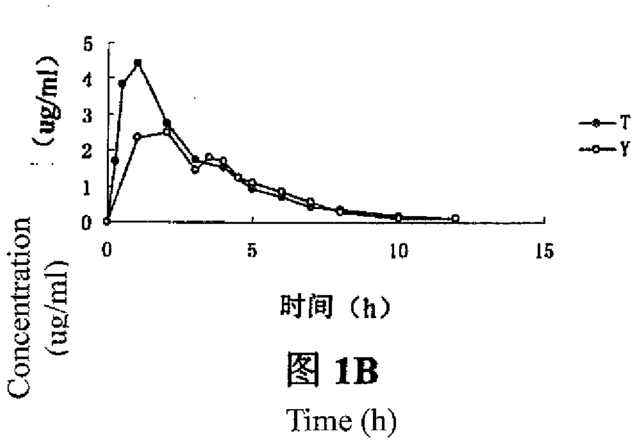


FIG. 1B

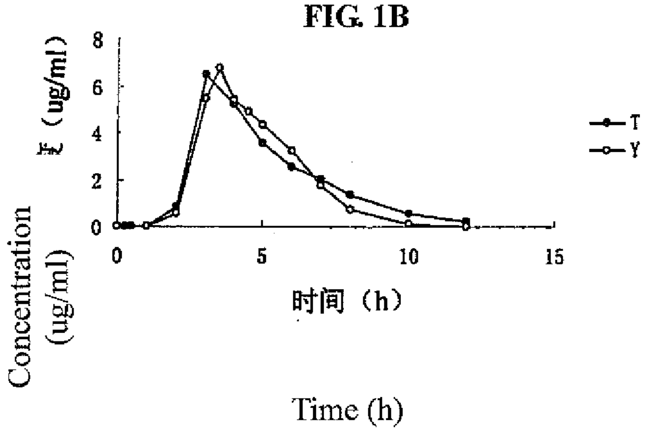


FIG. 1C

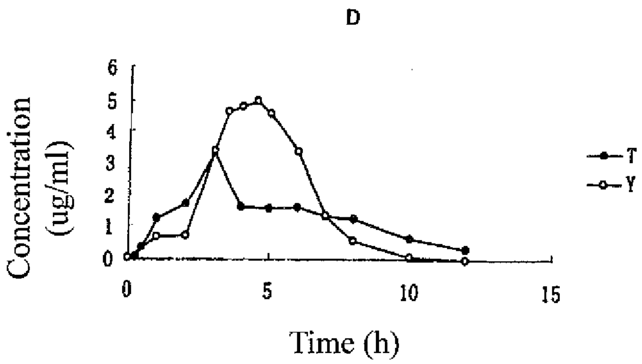


FIG. 1D

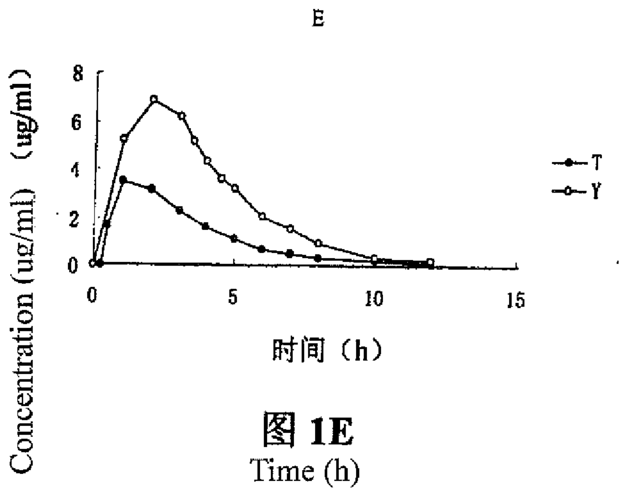


FIG. 1E

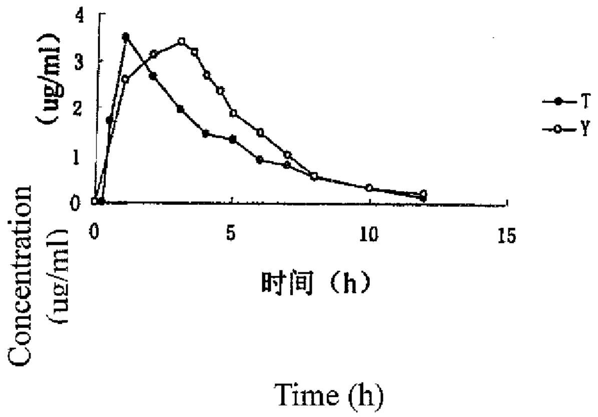


FIG. 1F

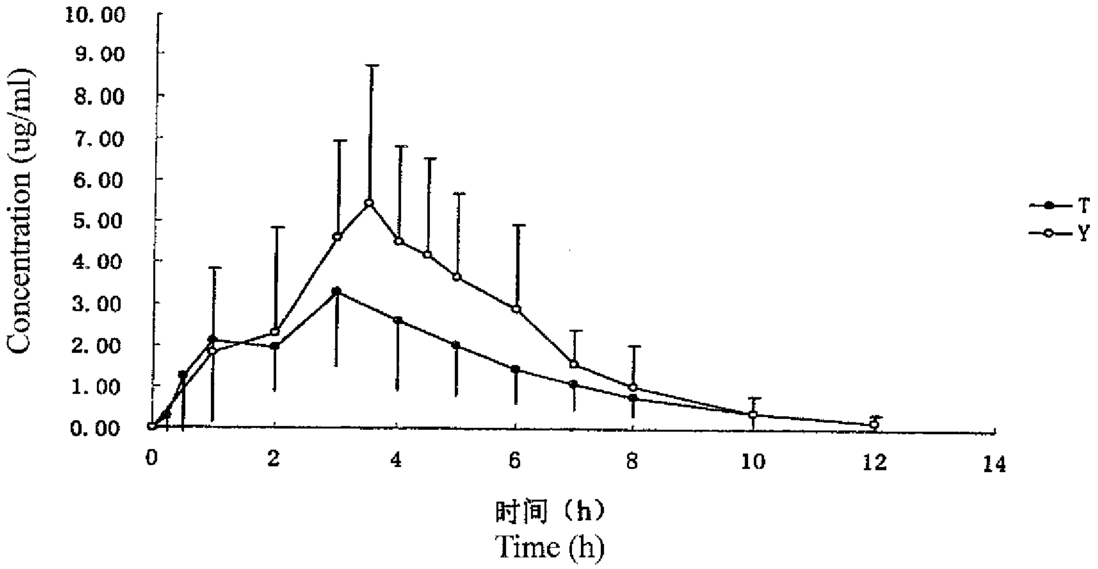
10.00
9.00
8.00
7.00
6.00
5.00
4.00
3.00
2.00
1.00
0.00
Concentration (ug/ml)
0
2
4
6
8
10
12
14
时间（h）
Time (h)
T
Y

FIG. 2

## INTERNATIONAL SEARCH REPORT

International application No.
PCT/CN2005/001545

A. CLASSIFICATION OF SUBJECT MATTER

IPC[7]: A61K31/4439, A61P1/04

According to International Patent Classification (IPC) or to both national classification and IPC

B. FIELDS SEARCHED

Minimum documentation searched (classification system followed by classification symbols)

WPI USPAT EPODOC PAJ CA MEDLINE

Documentation searched other than minimum documentation to the extent that such documents are included in the fields searched

CNPAT CNKI

Electronic data base consulted during the international search (name of data base and, where practicable, search terms used)

Benzimidazole pyridiminazole sulfinyl $C_{19}H_{23}N_3O_4S$ RN: 117976-53-1 +prazole

C. DOCUMENTS CONSIDERED TO BE RELEVANT

| Category* | Citation of document, with indication, where appropriate, of the relevant passages | Relevant to claim No. |
|---|---|---|
| P,X | WO2005054228 A1, (HETERO DRUGS LIMITED(US)), 16.JUN 2005, see claim 1 , formula lf and lf | 1-2, 6-9 |
| X | CN87107777A, (EISAI CO LTD (JP)), 1.JUN 1988, see pages 15-26,127, example 86 | 1-3, 6-9 |
| Y | | 4 |
| X | CA 117:3494 New fluorescent probes E3810 and methoxy E3810 for determining distributions of the apical membrane and the acidic compartment of gastric acid secreting cells , Takeguchi, etal, Jpn .J .Physiol., 42(1) ,see CA abstract | 1-3,6-9 |
| Y | | 4 |
| Y | CN1235018A(DONGYU PHARM CO LTD SHENYANG CITY(CN)) 17.NOV 1999, see whole document | 4 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim (S) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 22.NOV 2005(22.11.05) | 05 · JAN 2006 (05 · 01 · 2006) |
| Name and mailing address of the ISA/CN<br>The State Intellectual Property Office, the P.R.China<br>6 Xitucheng Rd., Jimen Bridge, Haidian District, Beijing, China 100088<br>Facsimile No. 86-10-62019451 | Authorized officer<br>WANG Jingjing 王晶晶印<br>Telephone No. 86-10-62085257 |

Form PCT/ISA /210 (second sheet) (April 2005)

**INTERNATIONAL SEARCH REPORT**
Information on patent family members

| International application No. |
|---|
| PCT/CN2005/001545 |

| Patent Documents referred in the Report | Publication Date | Patent Family | Publication Date |
|---|---|---|---|
| WO2005054228A1 | 16.06.2005 | ---------------- | |
| CN87107777A | 01.06.1988 | EP0268956 A | 01.06.1988 |
| | | AU8113887 A | 19.05.1988 |
| | | NO8704477 A | 06.06.1988 |
| | | DK8705758 A | 14.05.1988 |
| | | FI8704709 A | 14.05.1988 |
| | | HU45524 A | 28.07.1988 |
| | | JP1006270 A | 10.01.1989 |
| | | CA1265138 A | 30.01.1990 |
| | | US5045552 A | 03.09.1991 |
| | | DD290192 A | 23.05.1991 |
| | | NZ222488 A | 26.05.1993 |
| | | JP5247035 A | 24.09.1993 |
| | | FI90544B B | 15.11.1993 |
| | | DK9301318 A | 24.11.1993 |
| | | EP0268956 B1 | 06.04.1994 |
| | | DE3789536G G | 11.05.1994 |
| | | JP6074272B B2 | 21.09.1994 |
| | | ES2061471T T3 | 16.12.1994 |
| | | JP7291967 A | 07.11.1995 |
| | | DK9501442 A | 19.12.1995 |
| | | DK171024B B | 22.04.1996 |
| | | JP2544567B2 B2 | 16.10.1996 |
| | | JP2576843B2 B2 | 29.01.1997 |
| | | EP0268956 B2 | 15.04.1998 |
| | | US5840910 A | 24.11.1998 |
| | | US5998445 A | 07.12.1999 |
| | | DK174366B B | 13.01.2003 |
| CN1235018A | | ------------------ | |

Form PCT/ISA /210 (patent family annex) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 150586 A
- WO 921296 A
- WO 9324480 A
- WO 9602535 A